# EUROPEAN PATENT APPLICATION

(11) **EP 4 287 192 A1**
(43) Date of publication of application: **06.12.2023**
(21) Application number: 21923090.1
(22) Date of filing: 22.11.2021
(51) Int. Cl.: G16H 10/00, G06Q 10/10

(54) **DATA INPUT ASSISTANCE SYSTEM**

(30) Priority: 27.01.2021 JP 2021010909
(71) Applicant: CMIC Holdings Co. Ltd., Tokyo 105-0023 (JP)
(72) Inventor: FUKUSHI Gakuho, Tokyo 105-0023 (JP)
(74) Representative: Eisenführ Speiser
(86) International application number: PCT/JP2021/042713
(87) International publication number: WO 2022/163085

(57) **Abstract**

A data input assistance system (1) comprises a configuration data creation system (3) and an input data display system (2). The creation system (3) creates and outputs a configuration code (C1) obtained by encoding configuration data structured with a data name of actual data to be input to a data input terminal (10) and a control code name of a control code used at the time of inputting the actual data. The display system (2) creates and displays a data input code (C2) obtained by encoding the actual data to be input to the data input terminal, on the basis of the configuration data output from the configuration data creation system (3). When the data input terminal reads a data input code (C2), the actual data which has been encoded into the data input code (C2) is input to the data input terminal (10) by using the control code.

## Description

### Technical Field

The present invention is related to a data input assistance system provided with a function of assisting data input to a data input terminal.

### Background Art

Conventionally, electronic charts are used in medical facilities such as hospitals. Because the information included in electronic charts is extremely confidential, a terminal used for an electronic chart system is placed in an offline environment so as to prohibit wired/wireless data communication with other terminals. Conventionally, when information (e.g., data related to vaccination) from another terminal is transferred to such an electronic chart system, it is necessary to manually input data to the terminal used for the electronic chart system.

Aside from the above, systems for assisting data input at medical institutions and the like have conventionally been proposed. For example, a system has been proposed by which, when information written in a prescription is changed into a code, so that the code is read by a camera, drug name data is extracted from prescription data and displayed on a screen in a list format (see Patent Literature 1).

However, the conventional system is based on the assumption that data (the drug name data) is input to a predetermined single input form (an input form for the drug name data). Thus, compatibility with various types of input forms is not at all considered.

### Citation List

### Patent Literature

Patent Literature 1: Japanese Patent No. 5419244

### Summary of Invention

### Technical Problem

The present invention was conceived in view of the background described above. It is an object of the present invention to provide a data input assistance system compatible with various types of input forms.

### Solution to Problem

One aspect of the present invention provides a data input assistance system for inputting actual data recorded in an input data display system to a data input terminal. In the data input assistance system, the data input terminal uses an input form for the inputting of the actual data and uses a control code for confirming the input to the input form or for moving to another input form. The data input assistance system includes: a configuration data creation system that creates and outputs a configuration code obtained by encoding configuration data structured with a data name of the actual data to be input to the data input terminal and a control code name of the control code used at a time of inputting the actual data; and the input data display system that creates and displays a data input code obtained by encoding the actual data to be input to the data input terminal, on a basis of the configuration data output from the configuration data creation system. The configuration data creation system includes: a data selection unit that selects the data name of the actual data to be input to the data input terminal and the control code name of the control code used at the time of inputting the actual data; an order determination unit that determines a sequential order to arrange the data name and the control code name selected by the data selection unit; a configuration code generation unit that generates the configuration code obtained by encoding the configuration data in which the data name and the control code name selected by the data selection unit are arranged in the sequential order determined by the order determination unit; and a configuration code output unit that outputs the configuration code. The input data display system includes: an import unit that reads the configuration code output from the configuration data creation system, thereby performs a decoding process to obtain the configuration data which has been encoded into the configuration code, and imports the configuration data into the input data display system; a data input code generation unit that generates a data input code obtained by encoding input data in which the data name included in the configuration data is replaced with the actual data corresponding to the data name, and the control code name included in the configuration data is replaced with the control code corresponding to the control code name; and an input code display unit that displays the data input code. The data input terminal includes: a data input unit that reads the data input code displayed by the input data display system, thereby performs a decoding process to obtain the input data which has been encoded into the data input code, and inputs the actual data included in the input data to the data input terminal by using the control code.

Another aspect of the present invention provides an information input method implemented by a data input assistance system for inputting actual data recorded in an input data display system to a data input terminal. According to the information input method, the data input terminal uses an input form for the inputting of the actual data and uses a control code for confirming the input to the input form or for moving to another input form. The data input assistance system includes: a configuration data creation system that creates and outputs a configuration code obtained by encoding configuration data structured with a data name of the actual data to be input to the data input terminal and a control code name of the control code used at a time of inputting the actual data; and the input data display system that creates and displays a data input code obtained by encoding the actual data to be input to the data input terminal, on a basis of the configuration data output from the configuration data creation system. The method includes causing the configuration data creation system to perform: selecting the data name of the actual data to be input to the data input terminal and the control code name of the control code used at the time of inputting the actual data, determining a sequential order to arrange the data name and the control code name selected; generating the configuration code obtained by encoding the configuration data in which the data name and the control code name selected are arranged in the sequential order determined; and outputting the configuration code. The method further includes causing the input data display system to perform: reading the configuration code output from the configuration data creation system, thereby performing a decoding process to obtain the configuration data which has been encoded into the configuration code, and importing the configuration data into the input data display system; generating a data input code obtained by encoding input data in which the data name included in the configuration data is replaced with the actual data corresponding to the data name, and the control code name included in the configuration data is replaced with the control code corresponding to the control code name; and displaying the data input code. The method further includes causing the data input terminal to perform: reading the data input code displayed by the input data display system, thereby performing a decoding process to obtain the input data which has been encoded into the data input code, and inputting the actual data included in the input data to the data input terminal by using the control code.

As explained below, the present invention has other modes. Accordingly, the disclosure of the present invention is meant to provide a part of the modes of the present invention and does not intend to limit the scope of the invention described and claimed herein.

### Brief Description of Drawings

[Figure 1] Figure 1 is a drawing for explaining a data input assistance system according to the present embodiment.
[Figure 2] Figure 2 is a block diagram showing a configuration of a data input assistance system according to the present embodiment.
[Figure 3] Figure 3 is a drawing showing an example of a format of an electronic chart.
[Figure 4] Figure 4 is a table showing examples of data names, actual data, substitution data, control code names, and control codes.
[Figure 5] Figure 5 is a drawing showing an example of a screen for selecting data names and control code names.
[Figure 6] Figure 6 is a drawing showing examples of configuration data, verification data, and input data.
[Figure 7] Figure 7 is a drawing showing an example of a result of reading a verification code.
[Figure 8] Figure 8 is a drawing showing an example of a result of reading a data input code.
[Figure 9] Figure 9 is a sequence chart for explaining an operation of the data input assistance system according to the present embodiment.
[Figure 10] Figure 10 is a sequence chart for explaining the operation of the data input assistance system according to the present embodiment.

### Description of Embodiments

The following will describe the present invention in detail. However, the following detailed explanations and the accompanying drawings are not to limit the invention.

A data input assistance system according to the present invention is a data input assistance system for inputting actual data recorded in an input data display system to a data input terminal, wherein the data input terminal uses an input form for the inputting of the actual data and uses a control code for confirming the input to the input form or for moving to another input form, the data input assistance system includes: a configuration data creation system that creates and outputs a configuration code obtained by encoding configuration data structured with a data name of the actual data to be input to the data input terminal and a control code name of the control code used at a time of inputting the actual data; and the input data display system that creates and displays a data input code obtained by encoding the actual data to be input to the data input terminal, on a basis of the configuration data output from the configuration data creation system, the configuration data creation system includes: a data selection unit that selects the data name of the actual data to be input to the data input terminal and the control code name of the control code used at the time of inputting the actual data; an order determination unit that determines a sequential order to arrange the data name and the control code name selected by the data selection unit; a configuration code generation unit that generates the configuration code obtained by encoding the configuration data in which the data name and the control code name selected by the data selection unit are arranged in the sequential order determined by the order determination unit; and a configuration code output unit that outputs the configuration code, the input data display system includes: an import unit that reads the configuration code output from the configuration data creation system, thereby performs a decoding process to obtain the configuration data which has been encoded into the configuration code, and imports the configuration data into the input data display system; a data input code generation unit that generates a data input code obtained by encoding input data in which the data name included in the configuration data is replaced with the actual data corresponding to the data name, and the control code name included in the configuration data is replaced with the control code corresponding to the control code name; and an input code display unit that displays the data input code, and the data input terminal includes: a data input unit that reads the data input code displayed by the input data display system, thereby performs a decoding process to obtain the input data which has been encoded into the data input code, and inputs the actual data included in the input data to the data input terminal by using the control code.

With this arrangement, by using the data input code, it is possible to easily input the actual data stored in the input data display system to the data input terminal. It is possible to easily create the data input code by using the configuration code. It is possible to easily create the configuration code so as to be compatible with the input form used by the data input terminal. Consequently, even when the data input terminal uses various types of input forms, it is possible to easily achieve compatibility.

In the data input assistance system according to the present invention, the configuration data creation system may include: a verification code generation unit that, at a time of generating the configuration code, generates a verification code obtained by encoding verification data in which the data name included in the configuration data is replaced with substitution data corresponding to the data name, and the control code name included in the configuration data is replaced with the control code corresponding to the control code name; and a verification code display unit that displays the verification code, and the data input terminal may include: a substitution data display unit that reads the verification code displayed by the configuration data creation system, thereby performs a decoding process to obtain the verification data which has been encoded into the verification code, and displays the substitution data included in the verification data.

With this arrangement, when the configuration code is created, the verification code is created. By using the verification code, it is possible to easily verify whether or not the configuration code has been created as intended (so as to be compatible with the input form used by the data input terminal).

Further, in the data input assistance system according to the present invention, the data input terminal may be placed in an offline environment in relation to the configuration data creation system and the input data display system.

With this arrangement, by placing the data input terminal in the offline environment, it is possible to guarantee a high level of confidentiality.

Further, in the data input assistance system according to the present invention, the input data display system may include: a configuration data storage unit that stores therein a plurality of pieces of configuration data imported into the input data display system; and a form selection unit that selects the configuration data used for generating the data input code from among the plurality of pieces of configuration data stored in the configuration data storage unit.

With this arrangement, by selecting the configuration data to be actually used for inputting the data to the data input terminal from among the plurality of pieces of configuration data imported in advance, it is possible to easily achieve compatibility with various types of input forms.

An information input method of the present invention is an information input method implemented by a data input assistance system for inputting actual data recorded in an input data display system to a data input terminal, wherein the data input terminal uses an input form for the inputting of the actual data and uses a control code for confirming the input to the input form or for moving to another input form, the data input assistance system includes: a configuration data creation system that creates and outputs a configuration code obtained by encoding configuration data structured with a data name of the actual data to be input to the data input terminal and a control code name of the control code used at a time of inputting the actual data; and the input data display system that creates and displays a data input code obtained by encoding the actual data to be input to the data input terminal, on a basis of the configuration data output from the configuration data creation system, the method includes causing the configuration data creation system to perform: selecting the data name of the actual data to be input to the data input terminal and the control code name of the control code used at the time of inputting the actual data, determining a sequential order to arrange the data name and the control code name selected by the data selection unit; generating the configuration code obtained by encoding the configuration data in which the data name and the control code name selected by the data selection unit are arranged in the sequential order determined by the order determination unit; and outputting the configuration code, the method further includes causing the input data display system to perform: reading the configuration code output from the configuration data creation system, thereby performing a decoding process to obtain the configuration data which has been encoded into the configuration code, and importing the configuration data into the input data display system; generating a data input code obtained by encoding input data in which the data name included in the configuration data is replaced with the actual data corresponding to the data name, and the control code name included in the configuration data is replaced with the control code corresponding to the control code name; and displaying the data input code, and the method further includes causing the data input terminal to perform: reading the data input code displayed by the input data display system, thereby performing a decoding process to obtain the input data which has been encoded into the data input code, and inputting the actual data included in the input data to the data input terminal by using the control code.

Similarly to the system described above, according to this method, by using the data input code, it is possible to easily input the actual data stored in the input data display system to the data input terminal. It is possible to easily create the data input code by using the configuration code. It is possible to easily create the configuration code so as to be compatible with the input form used by the data input terminal. Accordingly, even when the data input terminal uses various types of input forms, it is possible to easily achieve compatibility.

According to the present invention, by using the configuration code and the data input code, it is possible to easily achieve the compatibility with various types of input forms.

### Embodiments

The following will describe a data input assistance system according to an embodiment of the present invention, with reference to the drawings. In the present embodiment, an example of a data input assistance system used in a vaccination history management system or the like will be explained. The data input assistance system is a system for inputting actual data recorded in an input data display system, to a data input terminal.

For example, the input data display system may be a vaccination history management system installed at a medical institution, whereas the data input terminal may be an electronic chart system installed at the medical institution. As explained later, the data input terminal uses an input form for inputting the actual data and uses a control code for confirming the input to the input form or for moving to another input form.

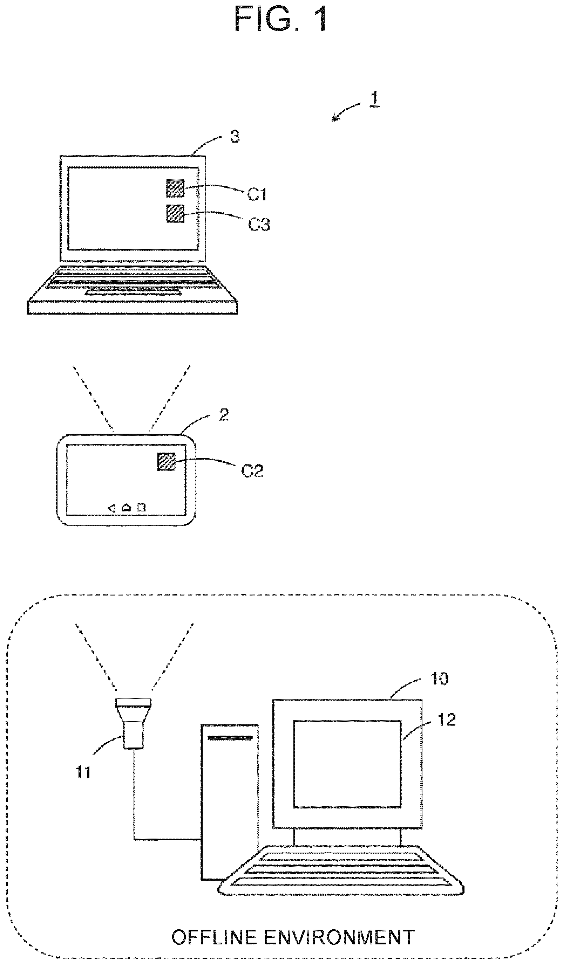

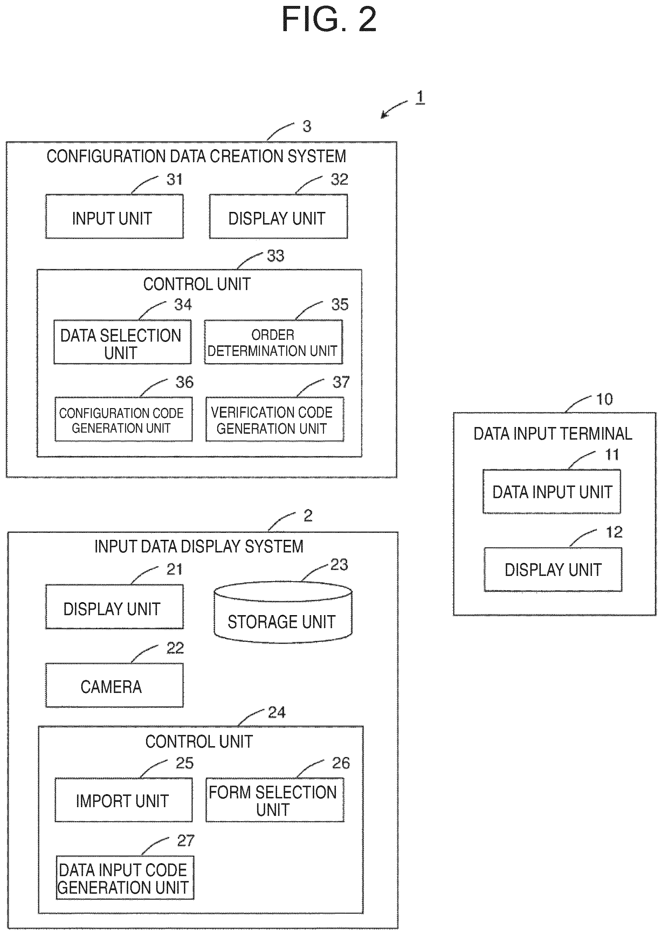

A configuration of the data input assistance system according to an embodiment of the present invention will be explained, with reference to the drawings. Figure 1 is a drawing for explaining the data input assistance system according to the present embodiment. Figure 2 is a block diagram showing a configuration of the data input assistance system according to the present embodiment. As shown in Figure 1 and Figure 2, the data input assistance system 1 includes an input data display system 2 and a configuration data creation system 3. In the following sections, an example will be explained in which the input data display system 2 and the configuration data creation system 3 are structured as separate devices; however, the input data display system 2 and the configuration data creation system 3 may be structured as a single device.

The configuration data creation system 3 is a system that creates and outputs a configuration code C1 obtained by encoding configuration data structured with a data name of actual data input to a data input terminal 10 and a control code name of a control code used at the time of inputting the actual data and is configured by using a personal computer or the like (see Figure 1), for example. The configuration code C1 is a two-dimensional code such as a QR code (registered trademark), for example. The functions of the configuration data creation system 3 described below may be provided in a cloud server or may be provided in a program installed in the host computer thereof.

Further, the input data display system 2 is a system that creates and displays a data input code C2 obtained by encoding the actual data to be input to the data input terminal 10, on the basis of the configuration data output from the configuration data creation system 3 and is configured by using a tablet terminal or the like (see Figure 1), for example. The data input code C2 is also a two-dimensional code such as a QR code, for example. The functions of the input data display system 2 described below are provided in a program installed in the host terminal thereof.

The configuration data creation system 3 includes an input unit 31, a display unit 32, and a control unit 33. For example, the input unit 31 is configured by using a keyboard or the like. The display unit 32 is configured by using a display or the like. The control unit 33 is configured by using a processor or the like and includes, as functional blocks thereof, a data selection unit 34, an order determination unit 35, a configuration code generation unit 36, and a verification code generation unit 37.

The data selection unit 34 has a function of selecting data names of the actual data to be input to the data input terminal 10 and the control code names of the control codes used at the time of inputting the actual data. The order determination unit 35 has a function of determining a sequential order to arrange the data names and the control code names selected by the data selection unit 34. The configuration code generation unit 36 has a function of generating the configuration code C1 obtained by encoding the configuration data in which the data names and the control code names selected by the data selection unit 34 are arranged in the sequential order determined by the order determination unit 35.

The verification code generation unit 37 has a function of generating, at the time of generating the configuration code C1, a verification code C3 obtained by encoding verification data in which the data names included in the configuration data are replaced with substitution data corresponding to the data names and the control code names included in the configuration data are replaced with the control codes corresponding to the control code names. The verification code C3 is also a two-dimensional code such as a QR code, for example. The display unit 32 has a function of displaying the configuration code C1 and the verification code C3.

The input data display system 2 includes a display unit 21, a camera 22, a storage unit 23, and a control unit 24. The display unit 21 has functions of both an input unit and a display unit. The camera 22 has a function of reading the configuration code C1 (e.g., a code reader function to read the QR code). The storage unit 23 is configured by using a memory or the like. The control unit 24 is configured by using a processor or the like and includes, as functional blocks thereof, an import unit 25, a form selection unit 26, and a data input code generation unit 27.

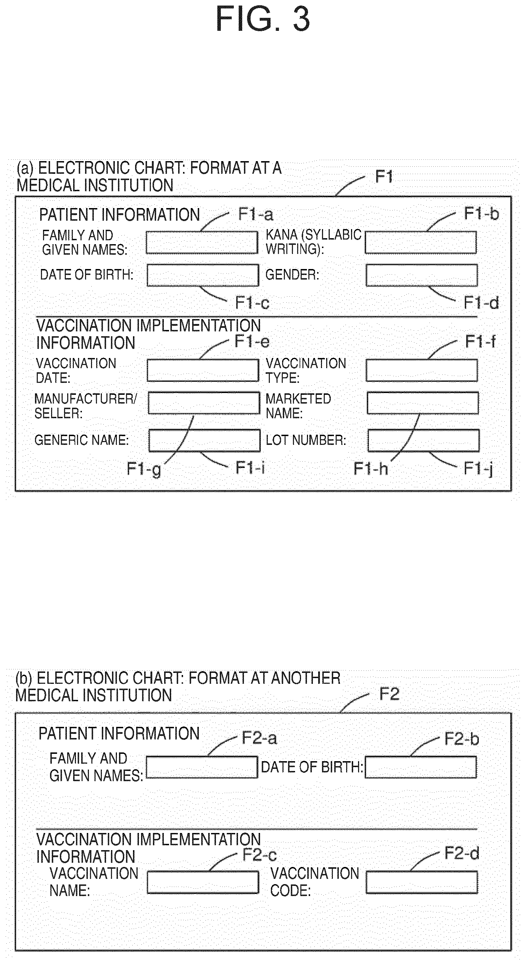

The import unit 25 has function of reading the configuration code C1 output from the configuration data creation system 3, thereby performing a decoding process to obtain the configuration data which has been encoded into the configuration code C1, and importing the configuration data to the input data display system 2. The storage unit 23 stores therein a plurality of pieces of configuration data imported into the input data display system 2. As explained later, the electronic chart uses various types of input forms F in correspondence with respective medical institutions (see Figure 3). The storage unit 23 is capable of storing therein various types of configuration data corresponding to the input forms F of the medical institutions.

The form selection unit 26 has a function of selecting the configuration data used for generating the data input code C2, from among the plurality of pieces of configuration data stored in the configuration data storage unit 23. The data input code generation unit 27 has the function of generating the data input code C2 obtained by encoding input data in which the data names included in the configuration data are replaced with the actual data corresponding to the data names and the control code names included in the configuration data are replaced with the control codes corresponding to the control code names. The display unit 21 has a function of displaying the data input code C2.

The data input terminal 10 includes a data input unit 11 and a display unit 12. The data input unit 11 has a function of reading the data input code C2 displayed by the input data display system 2, thereby performing a decoding process to obtain the input data which has been encoded into the data input code C2, and inputting the actual data to the data input terminal 10 by using the actual data and the control codes included in the input data. The data input unit 11 is configured by using a code reader or the like that reads the configuration code C1 and the verification code C3 (e.g., two-dimensional codes such as QR codes). The display unit 12 is configured by using a display device or the like, for example and has a function of displaying the actual data input to the data input terminal 10 by using the configuration code C1.

Further, the data input unit 11 has a function of reading the verification code C3 displayed by the configuration data creation system 3, thereby performing a decoding process to obtain the verification data which has been encoded into the verification code C3, and inputting the substitution data included in the verification data to the data input terminal 10. The display unit 12 has a function of displaying the substitution data input to the data input terminal 10 by using the verification code C3.

The configuration data creation system 3 and the input data display system 2 are placed in an offline environment (see Figure 1). Further, the data input terminal 10 may be configured by using an existing personal computer, for example. Further, the data input unit 11 may be a device separate from the data input terminal 10 or may be integrally formed with the data input terminal 10.

Next, the formats of the electronic chart will be explained. As shown in Figure 3, the data input terminal 10 uses various types of input forms. For instance, in the example of an electronic chart (the example of a format F1 at a certain medical institution) in Figure 3(a), an input form F1-a for inputting the actual data of the "Family and Given Names" of a patient who is to get vaccinated and another input form F1-b for inputting the actual data of "Kana (Japanese syllabic writing)" indicating how to read the family and given names are used. In addition, input forms F1-c to F1-j are used for inputting the actual data of "Date of Birth", "Gender", "Vaccination Date", "Vaccination Type", "Manufacturer/Seller", "Marketed Name", "Generic Name", "Lot number", and the like.

Further, in the example of an electronic chart (the example of a format F2 at another medical institution) in Figure 3(b), input forms F2-a to F2-d are used for inputting the actual data of "Family and Given Names", "Date of Birth", "Vaccination Name", "Vaccination Code", and the like. In this manner, the data input terminal 10 uses the various types of input forms, namely, F1-a to F1-j and F2-a to F2-d, in correspondence with the respective medical institutions. Further, as explained later, when a cursor is moved from an input form (e.g., F1-a) to another input form (e.g., F1-b), a control code (e.g., Tab) is used.

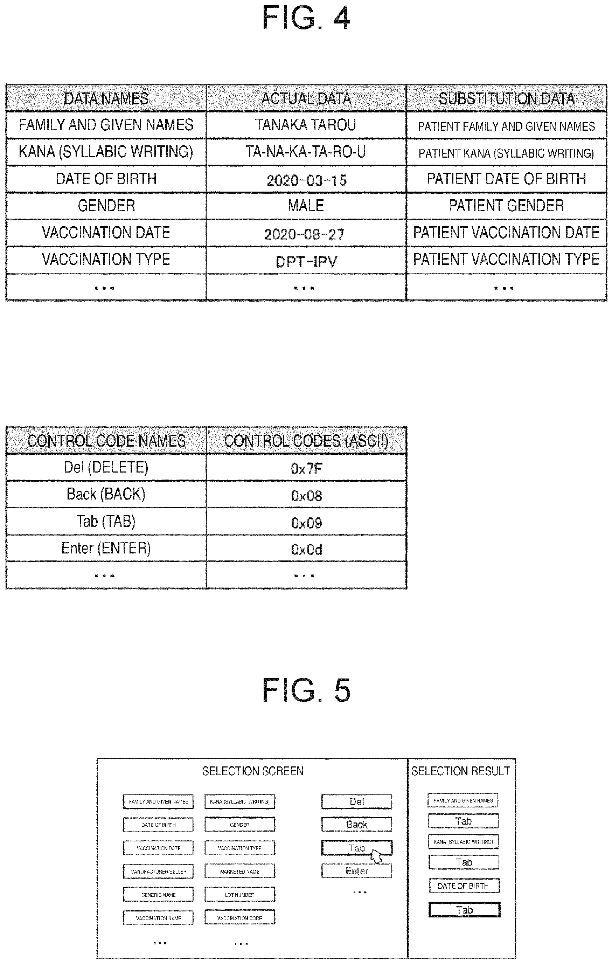

Next, the data names, the actual data, the substitution data, the control code names, and the control codes will be explained. As shown in Figure 4, the data names denote names such as "Family and Given Names" and "Gender" indicating the description of each data. The actual data denotes data in actuality such as "Tanaka Tarou" and "Male" recorded in the input data display system 2. The substitution data denotes dummy data such as "Patient Family and Given Names" and "Patient Date of Birth" that are used in place of the actual data for the purpose of verifying the inputs.

Further, the control code names denote the names of the control codes such as "Enter" and "Tab" used for confirming inputs to the input forms F or moving to a different one of the input forms F. The control codes denote actual control codes (ASCII codes) such as "0x0d" and "0x09".

Figure 5 is a drawing showing an example of a screen for selecting data names and control code names. As shown in Figure 5, on a selection screen (the frame on the left-hand side of Figure 5), when a cursor is placed over any of the "data names" and the "control code names" that are displayed, and a selecting operation (e.g., a click operation) is performed, the selected "data names" and "control code names" are displayed on a selection result screen (the frame on the right-hand side of Figure 5), while being arranged in the order in which the names were selected. On the selection result screen, it is possible to rearrange the order of the selected "data names" and "control code names" and to delete a selected item.

For example, in the format in Figure 3(a), the input forms F are arranged in the order of "Family and Given Names", "Kana (syllabic writing)", "Date of Birth", and so on. To achieve compatibility with this format, the data names and the control code names are selected in the order of "Family and Given Names", "Tab", "Kana (syllabic writing)", "Tab", "Date of Birth", "Tab", and so on, as shown in Figure 5.

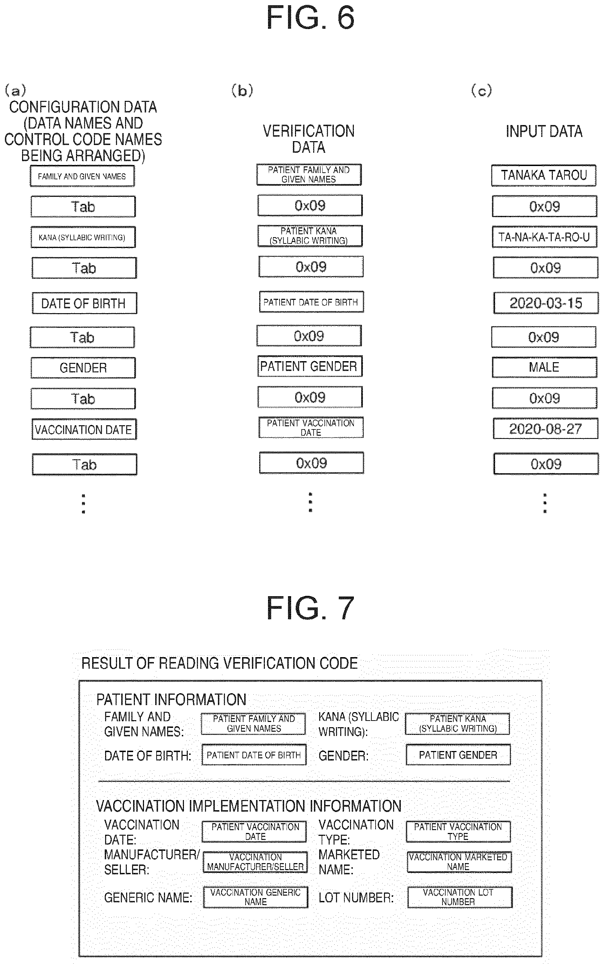

Figure 6 is a drawing showing examples of the configuration data, the verification data, and the input data. As shown in Figure 5, when the data names and the control code names are selected in the order of "Family and Given names", "Tab", "Kana (syllabic writing)", "Tab", "Date of Birth", "Tab", and so on, configuration data as shown in Figure 6(a) is created. On the basis of the configuration data in Figure 6(a), when the data names are replaced with the substitution data, and the control code names are replaced with the control codes, verification data as shown in Figure 6(b) is created. Further, on the basis of the configuration data in Figure 6(a), when the data names are replaced with the actual data, and the control code names are replaced with the control codes, input data as shown in Figure 6(c) is created.

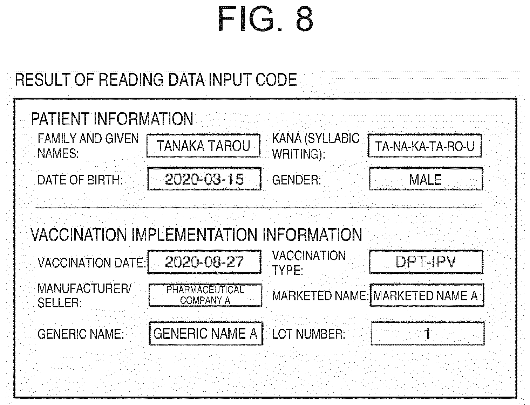

When the data input terminal 10 reads the verification code C3 obtained by encoding the verification data in Figure 6(b), the verification data decoded from the verification code C3 is displayed on the display unit 12, as shown in Figure 7. Further, when the data input terminal 10 reads the data input code C2 obtained by encoding the input data in Figure 6(c), the actual data decoded from the data input code C2 is input to the data input terminal 10 and displayed on the display unit 12, as shown in Figure 8.

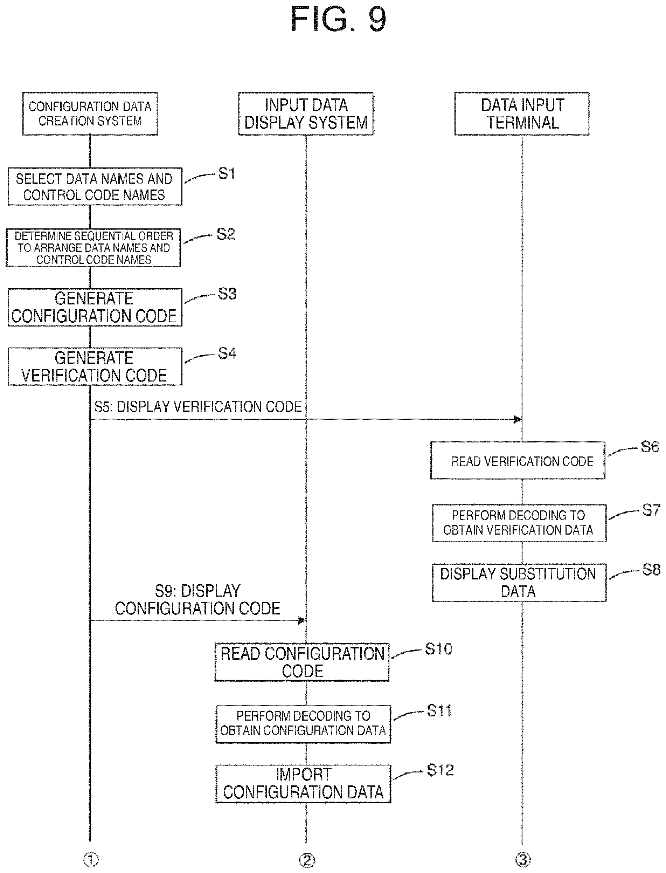

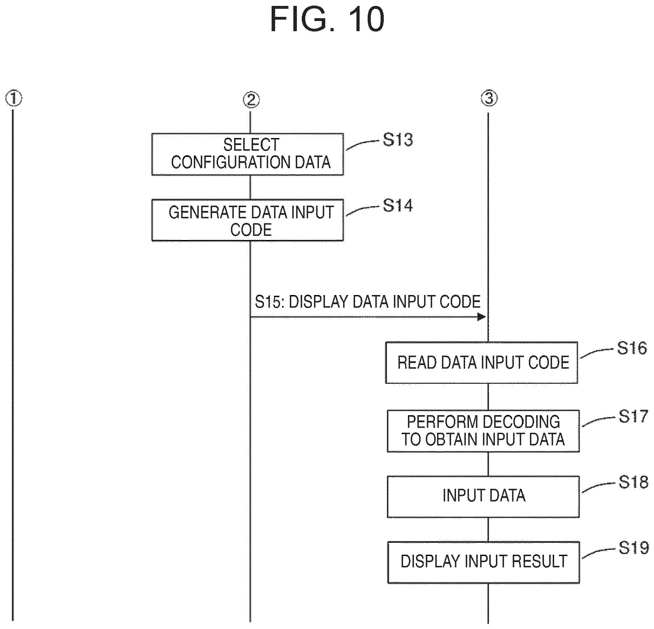

With regard to the data input assistance system 1 structured as described above, an operation thereof will be explained, with reference to the sequence charts in Figure 9 and Figure 10.

To assist the data input to the data input terminal 10 by using the data input assistance system 1 of the present embodiment, to begin with, the configuration data creation system 3 is used for selecting the data names of the actual data to be input to the data input terminal 10 and the control code names of the control codes to be used for the inputting of the actual data (S1), and further for determining the sequential order to arrange the selected data names and control code names (S2).

As a result, the configuration code C1 is generated by encoding the configuration data in which the data names and the control code names selected in step S1 are arranged in the sequential order determined in step S2 (S3). Further, the verification code C3 is generated by encoding the verification data in which the data names included in the configuration data are replaced with the substitution data, and the control code names are replaced with the control codes (S4).

When the configuration data creation system 3 displays the generated verification code C3 (S5), and the data input terminal 10 reads the verification code C3 (S6), the verification data which has been encoded into the verification code C3 is obtained through a decoding process (S7), and the substitution data included in the verification data is displayed on the display unit 12 (S8) .

Further, when the configuration data creation system 3 displays the generated configuration code C1 (S9), and the input data display system 2 reads the configuration code C1 (S10), the configuration data which has been encoded into the configuration code C1 is obtained through a decoding process (S11), and the configuration data is imported into the input data display system 2 (S12).

Subsequently, when the input data display system 2 is used for selecting the configuration data (S13), the data input code C2 is generated by encoding the input data in which the data names included in the configuration data are replaced with the actual data, and the control code names are replaced with the control codes (S14).

When the input data display system 2 displays the generated data input code C2 (S15), and the data input terminal 10 reads the data input code C2 (S16), the input data which has been encoded into the data input code C2 is obtained through a decoding process (S17), the actual data included in the input data is input to the data input terminal 10 (S18), and an input result thereof is displayed on the display unit 12 (S19).

In the data input assistance system 1 in the present embodiment as described above, by using the data input code C2, it is possible to easily input the actual data stored in the input data display system 2 to the data input terminal 10. It is possible to easily create the data input code C2 by using the configuration code C1. It is possible to easily create the configuration code C1 so as to be compatible with the input forms F used by the data input terminal 10. Consequently, even when the data input terminal 10 uses the various types of input forms F, it is possible to easily achieve the compatibility.

Further, in the present embodiment, when the configuration code C1 is created, the verification code C3 is created. By using the verification code C3, it is possible to easily verify whether or not the configuration code C1 has been created as intended (so as to be compatible with the input forms F used by the data input terminal 10).

Further, in the present embodiment, by placing the data input terminal 10 in the offline environment, it is possible to guarantee high confidentiality.

Furthermore, in the present embodiment, by selecting the configuration data actually used for the inputting of the data to the data input terminal 10 from among the plurality of pieces of configuration data imported in advance, it is possible to easily achieve the compatibility with the various types of input forms F.

The embodiments of the present invention have thus been explained with the examples. However, the scope of the present invention is not limited to these examples and may be changed or modified in accordance with purposes, within the scope set forth in the claims.

For example, although the above description explains the example in which the configuration data creation system 3 displays the configuration code C1, and the input data display system 2 reads the configuration code C1, the scope of the present invention is not limited to this example. When the configuration data creation system 3 and the input data display system 2 are capable of performing data communication, the configuration data creation system 3 may output (transmit) the configuration code C1, and the input data display system 2 may receive the input (receive the transmission) of the configuration code C1.

Further, although the above description explains the example in which the data input assistance system 1 of the present invention is applied to the vaccination history management system (the example in which the input data display system 2 is the vaccination history management system installed at a medical institution, whereas the data input terminal 10 is the electronic chart system installed at the medical institution), the scope of the present invention is not limited to this example.

The data input assistance system 1 of the present invention may be applied to an electronic medication handbook system. In that situation, the input data display system 2 may be an electronic medication handbook system installed at a pharmacy, whereas the data input terminal 10 may be a reception computer terminal or a terminal having a medication history management function installed at the pharmacy.

Further, the data input assistance system 1 of the present invention may be applied to a questionnaire registration system at a hospital. In that situation, the input data display system 2 may be a smartphone owned by a patient, whereas the data input terminal 10 may be an electronic chart system installed at a medical institution.

Further, the data input assistance system 1 of the present invention may be applied to any of various types of administrative procedure systems for address registration or the like used at public institutions (including government offices, notary offices, courts, police stations, and other administrative institutions). In that situation, the input data display system 2 may be a personal information management system installed in a smartphone owned by a user or at a public institution, whereas the data input terminal 10 may be a resident information management terminal installed at the public institution.

Further, the data input assistance system 1 of the present invention may be applied to a system for transferring disability certificate information. In that situation, the input data display system 2 may be a disability certificate information management system installed in a smartphone owned by a user or at a medical institution, whereas the data input terminal 10 may be an electronic chart system installed at the medical institution.

Further, the data input assistance system 1 of the present invention may be applied to an address registration system at financial institutions including post offices, banks, and the like. In that situation, the input data display system 2 may be an address information management system installed in a smartphone owned by a user or at a financial institution, whereas the data input terminal 10 may be an information management terminal installed at the financial institution.

Further, the data input assistance system 1 of the present invention may be applied to a membership registration system in a store such as a clothing store or a rental video store. In that situation, the input data display system 2 may be an information management terminal installed in a smartphone owned by a user or at a store, whereas the data input terminal 10 may be an information management terminal installed at the store.

Further, the data input assistance system 1 of the present invention may be applied to a system for moving information between devices such as a tablet and a personal computer in an environment that is not connected to the Internet. In that situation, the input data display system 2 may be a terminal owned by a user, whereas the data input terminal 10 may be another terminal owned by the user.

Further, the data input assistance system 1 of the present invention may be applied to a system for transferring test data from a blood test or the like or medical examination data at a medical institution into an electronic chart. In that situation, the input data display system 2 may be a terminal for managing the test data or the medical examination data installed at the medical institution such as a measurement device installed at the medical institution, whereas the data input terminal 10 may be an electronic chart system installed at the medical institution.

Further, the data input assistance system 1 of the present invention may be applied to a system for transferring test data from a blood test or the like or medical examination data at a medical institution to a smartphone or a tablet owned by a user. In that situation, the input data display system 2 may be an information management terminal installed at the medical institution, whereas the data input terminal 10 may be the smartphone or the tablet owned by the user.

### Industrial Applicability

As explained above, the data input assistance system according to the present invention has an advantageous effect where the compatibility with the various types of input forms is easily achieved, and the system is thus useful as being applicable to a vaccination history management system or the like.

### Reference Signs List

- 1: data input assistance system
- 2: input data display system
- 3: configuration data creation system
- 10: data input terminal
- 11: data input unit
- 12: display unit
- 21: display unit
- 22: camera
- 23: storage unit
- 24: control unit
- 25: import unit
- 26: form selection unit
- 27: data input code generation unit
- 31: input unit
- 32: display unit
- 33: control unit
- 34: data selection unit
- 35: order determination unit
- 36: configuration code generation unit
- 37: verification code generation unit
- C1: configuration code
- C2: data input code
- C3: verification code
- F: input form

## Claims

1. A data input assistance system for inputting actual data recorded in an input data display system to a data input terminal, wherein
the data input terminal uses an input form for the inputting of the actual data and uses a control code for confirming the input to the input form or for moving to another input form,
the data input assistance system comprises:
a configuration data creation system that creates and outputs a configuration code obtained by encoding configuration data structured with a data name of the actual data to be input to the data input terminal and a control code name of the control code used at a time of inputting the actual data; and
the input data display system that creates and displays a data input code obtained by encoding the actual data to be input to the data input terminal, on a basis of the configuration data output from the configuration data creation system,
the configuration data creation system comprises:
a data selection unit that selects the data name of the actual data to be input to the data input terminal and the control code name of the control code used at the time of inputting the actual data;
an order determination unit that determines a sequential order to arrange the data name and the control code name selected by the data selection unit;
a configuration code generation unit that generates the configuration code obtained by encoding the configuration data in which the data name and the control code name selected by the data selection unit are arranged in the sequential order determined by the order determination unit; and
a configuration code output unit that outputs the configuration code,
the input data display system comprises:
an import unit that reads the configuration code output from the configuration data creation system, thereby performs a decoding process to obtain the configuration data which has been encoded into the configuration code, and imports the configuration data into the input data display system;
a data input code generation unit that generates a data input code obtained by encoding input data in which the data name included in the configuration data is replaced with the actual data corresponding to the data name, and the control code name included in the configuration data is replaced with the control code corresponding to the control code name; and
an input code display unit that displays the data input code, and
the data input terminal comprises:
a data input unit that reads the data input code displayed by the input data display system, thereby performs a decoding process to obtain the input data which has been encoded into the data input code, and inputs the actual data included in the input data to the data input terminal by using the control code.

2. The data input assistance system according to claim 1, wherein
the configuration data creation system comprises:
a verification code generation unit that, at a time of generating the configuration code, generates a verification code obtained by encoding verification data in which the data name included in the configuration data is replaced with substitution data corresponding to the data name, and the control code name included in the configuration data is replaced with the control code corresponding to the control code name; and
a verification code display unit that displays the verification code, and
the data input terminal comprises:
a substitution data display unit that reads the verification code displayed by the configuration data creation system, thereby performs a decoding process to obtain the verification data which has been encoded into the verification code, and displays the substitution data included in the verification data.

3. The data input assistance system according to claim 1 or 2, wherein
the data input terminal is placed in an offline environment in relation to the configuration data creation system and the input data display system.

4. The data input assistance system according to any one of claims 1 to 3, wherein
the input data display system comprises:
a configuration data storage unit that stores therein a plurality of pieces of configuration data imported into the input data display system; and
a form selection unit that selects the configuration data used for generating the data input code from among the plurality of pieces of configuration data stored in the configuration data storage unit.

5. An information input method implemented by a data input assistance system for inputting actual data recorded in an input data display system to a data input terminal, wherein
the data input terminal uses an input form for the inputting of the actual data and uses a control code for confirming the input to the input form or for moving to another input form,
the data input assistance system comprises:
a configuration data creation system that creates and outputs a configuration code obtained by encoding configuration data structured with a data name of the actual data to be input to the data input terminal and a control code name of the control code used at a time of inputting the actual data; and
the input data display system that creates and displays a data input code obtained by encoding the actual data to be input to the data input terminal, on a basis of the configuration data output from the configuration data creation system,
the method comprises causing the configuration data creation system to perform:
selecting the data name of the actual data to be input to the data input terminal and the control code name of the control code used at the time of inputting the actual data;
determining a sequential order to arrange the data name and the control code name selected;
generating the configuration code obtained by encoding the configuration data in which the data name and the control code name selected are arranged in the sequential order determined; and
outputting the configuration code,
the method further comprises causing the input data display system to perform:
reading the configuration code output from the configuration data creation system, thereby performing a decoding process to obtain the configuration data which has been encoded into the configuration code, and importing the configuration data into the input data display system;
generating a data input code obtained by encoding input data in which the data name included in the configuration data is replaced with the actual data corresponding to the data name, and the control code name included in the configuration data is replaced with the control code corresponding to the control code name; and
displaying the data input code, and
the method further comprises causing the data input terminal to perform:
reading the data input code displayed by the input data display system, thereby performing a decoding process to obtain the input data which has been encoded into the data input code, and inputting the actual data included in the input data to the data input terminal by using the control code.
